# EUROPEAN PATENT SPECIFICATION

(11) **EP 4 608 276 B1**
(45) Date of publication and mention of the grant of the patent: **14.10.2026**
(21) Application number: 23798151.9
(22) Date of filing: 24.10.2023
(51) Int. Cl.: A61B 8/08, A61B 8/12

(54) **MEDICAL APPARATUS FOR NATURAL ORIFICES**
MEDIZINISCHE VORRICHTUNG FÜR NATÜRLICHE ÖFFNUNGEN
APPAREIL MÉDICAL POUR ORIFICES NATURELS

(30) Priority: 28.10.2022 IT 202200022317
(43) Date of publication of application: 03.09.2025
(73) Proprietor: ELESTA S.P.A., 50041 Calenzano (FI) (IT)
(72) Inventor: MASOTTI, Giovanni, 50041 Calenzano (FI) (IT); BRESCHI, Luca, 59021 Vaiano (PO) (IT)
(74) Representative: Mannucci, Michele
(86) International application number: PCT/EP2023/079653
(87) International publication number: WO 2024/089033

(56) References cited:
- WO-A1-2016/135594
- US-A1- 2010 016 710

## Description

### Technical field

The present invention relate to medical equipment and its methods of use. More in particular, described herein are innovations in the sector of ultrasonic imaging equipment in the field of surgical interventions in particular, but not exclusively, on the prostate, for example transperineal approaches, or interventions associated with the vaginal region, for example through a transvaginal approach. There can be other possible uses in other regions, such as the abdomen.

### Background art

As is known, in the field of image diagnostics linked to treatments or surgical operations, it may be necessary to insert an endocavitary probe into a natural orifice of the body of a patient, for example the anus (for example through a transperineal approach) or the vagina (for example through a transvaginal approach), with association with a system of operative needles that can have different functions, for example that of performing biopsies or performing laser treatments.

For example, in interstitial laser therapy applied to the prostate with a transperineal approach using ultrasonic equipment, the aim is to thermally destroy a neoplastic part that has grown with respect to the physiological structure.

This can, for example, be represented by a benign prostatic hyperplasia that generates a type of symptoms such as frequent urination, burning, discomfort during urination and not, etc., generally indicated with the acronym LUTS (Lower Urinary Tract Symptoms).

The problems are often linked to the growth of the prostate lobes causing compression of the urethra along its course from the base to the apex of the prostate. The laser treatment also defined as LICR (*Laser induced cytoreduction*) determines a reduction in the volume of the treated part that decompresses the prostatic urethra until remission of the symptoms.

In some cases, with suitable ultrasound equipment, it is also possible to act on a malignant lesion of the prostate. Although the number of malignant lesions that can be displayed with ultrasound is relatively low, today there are high frequency ultrasound machines with improved image performance that allow real time display and consequently treatment of the lesion.

Current intervention techniques involve monitoring the area of intervention by means of ultrasound imaging techniques, displaying on a monitor, during the intervention, the positioning of optical fibers for lasers that are carried to the area of intervention by guide needles made to pass through the perineum of the patient.

An aim of this surgical technique is to remove all the benign or malignant tissue, maintaining the fundamental anatomical structures around it intact. Therefore, it is necessary to destroy portions of anatomical structure around the tumor to avoid leaving the slightest trace of benign or malignant neoplastic cells in the structure involved, without however eliminating too much of the anatomical structure, in order to allow functional preservation of the organ involved and of the adjacent structures.

Similar considerations can be made, for example, for interventions in the vaginal region, for example by means of a transvaginal approach.

Excellent control of the surgical and vision instruments of the area on which to act is crucial in order to reduce the risk of errors.

Prior art systems and methods for assisted surgical vision are disclosed in WO 2016/135594 A1 and US 2010/016710 A1.

### Summary

The object of the present invention is therefore to improve the aspects linked to operative interventions by means of the use of endocavitary probes in natural orifices, which makes it possible to improve the vision in the area in which to operate.

Another important object of the present invention is to improve the aspects linked to operative interventions by means of the use of endocavitary probes in natural orifices, which makes it possible to improve the precision of the intervention.

Yet another object of the present invention is, for example, to produce a prostate tissue-destructive radiation emission apparatus that makes it possible to suitably plan the areas to be destroyed.

One more important object of the present invention is, for example, to produce a prostate tissue-destructive radiation emission apparatus that makes it possible only to destroy the preselected anatomical structures.

A further object of the present invention is to develop a method of processing images that makes it possible to plan and forecast the result of an intervention to remove benign or malignant tumor cells, for example a surgical intervention on the prostate.

These and other objects, which will be more apparent below, are achieved with a medical apparatus using an ultrasound approach through natural orifices comprising
- an ultrasonic probe adapted to be introduced into a natural orifice of a body, having a principal axis, configured to acquire a spatial volume by acquiring scans along at least one scan plane associated with said principal axis;
- a vision system comprising a monitor on which at least one first image relating to an image in a plane transverse to said principal axis is visible;
- a first support system, configured to support the ultrasonic probe in the area to be investigated, wherein this first support system is adapted to constrain the probe to allow two movements, respectively a first axial movement in which the probe is moved along its principal axis and a second movement of rotation around this principal axis, the position of the probe in said first support system being known;
- position indicators of rotational position around the axis of said probe in the first support system and of position along the principal axis of the probe in the first support system;
- at least one operative needle adapted to be introduced into a body portion;
- a second support system, configured to support said at least one operative needle and to allow its movement along a trajectory, towards the area of intervention in which it is adapted to operate; this second support system is spatially related to the first support system for which the support position of the operative needle is known with respect to the ultrasonic probe, and with respect to the spatial volume investigated by the ultrasonic probe;
- an electronic device for managing the apparatus, comprising a correction module of the at least one first image visible on the monitor, adapted to rotate said at least one first image on the monitor in a manner concordant with a rotary movement of the probe around its principal axis, so that the content of said at least one first image relating to the area to be treated is fixed on the monitor during rotation of the probe.

The term concordant is meant as a rotation of the first image on the monitor such that the content of this image that is visible on the monitor is fixed, or steady, on the monitor, even if the probe rotates around the axis. For example, if the probe is positioned with its axis orthogonal to the screen of the monitor, and the probe is rotated clockwise (looking at the monitor), then the information relating to the image is rotated, substantially in real time, concordantly, i.e., clockwise looking at the monitor, around a point that corresponds to the principal axis of the probe, so that the content of the image is fixed on the monitor.

This is possible thanks to the fact that the angular positions, or the angular variations of the probe around its principal axis are known through the rotational position indicators and, therefore, the angular variation θ of the probe with respect to this axis corresponds to an angular correction θ of the image around the point corresponding to the axis of the probe on the monitor .

For example, considering each point or pixel of the first image on the monitor, given the angular coordinates x,y of the point on a Cartesian reference system orthogonal to the principal axis of the probe and the origin of which is coincident on said principal axis, the coordinate correction on the abscissa and on the ordinate, i.e., the new coordinates, will be x ' = x cos θ - y sin θ and y ' = x sin θ + y cos θ.

The apparatus according to the invention is well suited for interaction with human orifices, for example for interaction in the prostate or vaginal region. For example, the apparatus is well suited for the treatment of diseases of the prostate by means of laser ablation. Therefore, the ultrasonic probe is preferably an endocavitary probe, for example of the transrectal or endorectal type, i.e., a transrectal or endorectal ultrasound probe. The probe can also be of the transvaginal type, to be able to treat fibroids or tumors in this area.

Preferably, the scan plane is a plane transverse to the principal axis of the probe, and the at least one first image (which relates to an image along a plane transverse to the principal axis) comes from at least one scan of the probe in this plane transverse to the principal axis.

The term transverse is meant as a direction such that the plane is incident on the principal axis of the probe. Preferably, this is meant as a direction orthogonal to the axis of the probe.

Alternatively, the scan plane is a plane longitudinal to the principal axis of the probe, and wherein the at least one first image relating to an image in a plane transverse to the principal axis is reconstructed from a plurality of scans performed in a plane longitudinal to this principal axis, for example a star of planes passing through this principal axis.

The term longitudinal is meant as a direction such that the plane is parallel to the principal axis (i.e., is not incident on this plane) or a direction such that the principal axis lies completely on the plane. Hereunder the term "parallel" may be used equivalently to the term "longitudinal" described above.

Hereunder, the term "tissue" or "tissues" is meant as any portion of the human anatomical structure.

Preferably, on the monitor are provided at least one second image relating to a scan of the probe in at least one plane longitudinal to the principal axis.

Therefore, on the monitor preferably are provided at least two images, a first image relating to a view in a plane "transverse", preferably orthogonal, to the principal axis of the probe, for example produced by means of a scan on the plane transverse to (or a view on a plane transverse to the axis but produced through the combination of a plurality of scans longitudinal to the principal axis, the points of which relating to a same transverse plane are combined to show a view on this plane) the axis of the probe, and a second image relating to a plane longitudinal to the axis of the probe. The view of the two images (transverse and longitudinal) allows a three-dimensional view of the area investigated by the probe on the monitor. A third image can also be present, showing the area investigated from a third plane orthogonal to the other two, not scanned but reconstructed by means of the information of the other two images.

Therefore, to this end, the ultrasonic probe can preferably comprise an oblong body, with a convex curved outer surface, extending along a longitudinal development of this body, wherein the body is provided with a plurality of ultrasonic sensors facing out on the curved surface for emitting and receiving ultrasonic waves; preferably the curved surface of the ultrasonic probe is substantially cylindrical and has a longitudinal axis parallel to the longitudinal development of the oblong body; preferably, the ultrasonic sensors of the probe are arranged according to at least one rectilinear curtain, parallel to the axis of the probe and at least one curvilinear curtain, lying on a circumference in a plane orthogonal to the axis of the probe. According to an embodiment, the ultrasonic sensors of the probe are arranged according to a single rectilinear curtain, parallel to the axis of the probe and a single curvilinear curtain, lying on a circumference in a plane orthogonal to the axis of the probe.

Advantageously, the first support system is adapted to constrain the probe to permit only the aforesaid two movements: the first axial movement in which the probe is moved along its principal axis and the second movement of rotation around said principal axis, thereby allowing determination of the position of the probe to be simplified.

In preferred embodiments, in view of the most widely hypothesized uses, the ultrasonic probe is an endocavitary ultrasonic probe, preferably of transrectal type or of transvaginal type.

Advantageously, according to preferred embodiments, the at least one operative needle is a guide needle, movable along a trajectory defined at least in part by the second support system. For example, this guide needle can carry laser light emitting elements, or more generally devices emitting tissue-destructive beams, devices for performing biopsies, video cameras, etc.

Preferably, at least one tissue-destructive radiation emission head is arranged in this at least one guide needle, so that this needle is configured to support the emission head and allow its movement along a trajectory, towards the area to be treated.

For example, the at least one tissue-destructive radiation emission head is a member adapted to emit a laser beam; preferably the end of an optical fiber. Therefore, the apparatus of the invention also comprises a radiation source, for example a laser light source with which the optical fiber is associated.

The apparatus is also well suited for tissue-destructive treatment in various areas of the body, including the transperineal, transvaginal and abdomen area.

According to preferred embodiments, the electronic device for managing the apparatus comprises an electronic program equipped with a tissue destruction simulation module which provides for the operations of
spatial definition, on at least said first image, of the area of intervention where the destructive treatment is to be performed;
display of a simulation of the virtual position of at least one said emission head in said area of intervention on said at least one first image shown on a monitor;
preferably the electronic program provides for the display on said at least one image on said monitor, of at least one virtual trajectory developing from the support position for said at least one emission head on said second support system, wherein said virtual trajectory defines a path for said emission head towards the virtual position in the area of intervention.

Preferably, the electronic program provides for the display, on the at least one first image shown on a monitor, of a simulation of the volume that can be irradiated by the at least one emission head positioned virtually in the area of intervention, and wherein the size of the volume that can be irradiated by the at least one emission head positioned virtually in the area of intervention is a function of one or more of the following parameters: power of the emitted radiation, the amount or dose of energy of the emitted radiation, any movement of the at least one emission head during the emission phase, the length of said possible movement, the Arrhenius damage value.

Preferably, the simulation operations of the simulation module are also performed on the second image relating to a scan of said probe in at least one plane longitudinal to the principal axis.

Preferably, the electronic program comprises a destructive treatment module of the area to be treated, which provides for the operation of setting treatment parameters, corresponding to virtual treatment parameters set in the apparatus during operation of said simulation module, said parameters comprising one or more of the following: number of emission heads positioned at the emission zone, position of the at least one emission head in the emission zone, power of the radiation emitted by the at least one emission head, the amount or dose of energy of the radiation emitted by the at least one emission head, any movement of the at least one emission head during the emission phase, the length of said possible movement.

Preferably, the simulation module provides for
real time calculation of the simulation on the basis of one or more of the following parameters: number of ablation heads, mutual positioning of the ablation heads, dose of energy applied by each ablation head, power of the ablation radiation source, possible presence of pullback actions, with definition of any pullback length/distance, or
a database of previous simulations performed on the basis of one or more of the following parameters: number of ablation heads, mutual positioning of the ablation heads, dose of energy applied by each ablation head, power of the ablation radiation source, possible presence of pullback actions, with definition of any pullback length/distance, so that the surgeon can retrieve the results of a previously performed simulation with the same operating parameters indicated above, saving calculation time.

Returning to the rotation of the at least one first ultrasound image, this represents a novelty in the ultrasound sector. Currently, ultrasound scanners produce a fixed image on the screen independent of the angle of rotation of the probe. This creates a movement of the anatomical structures within the image during rotation of the probe. According to the invention, the rotation of the image linked to the rotation of the probe produces invariance of the anatomical structures, which remain spatially correlated with the patient. This makes the planning process more intuitive, reducing risks of error.

As already mentioned, the ultrasonic probe reports images of the ultrasonic volume investigated in real time; these images can be three-dimensional or flat images, for example along the sagittal, coronal and transverse planes (which combined with one another allow the reconstruction of a three-dimensional image).

Therefore, the electronic program of the apparatus is preferably able to manage both several images of different planes, and one or more three-dimensional images. For example, the two-dimensional images are two-dimensional images of corresponding sagittal, coronal and transverse planes.

The ultrasonic probe is inserted into the rectum of the patient. This probe is associated with a said first support system, which, is preferably also configured to guide the ultrasonic probe in the rectum. Advantageously, the first support system allows the position of the ultrasonic probe in the first support system, i.e., in space, to be known at all times. For example, the probe is associated with a reference system fixed with respect to the patient, for example fixed with respect to the patient's bed, i.e., with a support for the probe fixed with respect to the bed in which the patient is lying.

Once the probe has allowed an ultrasonic volume of the areas to be treated to be acquired, which translates into the acquisition of at least a first image (for example at least two or three two-dimensional images relative for example to the sagittal, coronal and transverse plane, or to a combination of the three planes to produce at least one three-dimensional image), the intervention planning module is launched.

For example, in the case of a tissue-destructive intervention, the planning module provides for an operation of graphically circumscribing the area with the tissue to be destroyed before combining the at least one first and at least one second image. For example, the area with the tumor (or other formation to be destroyed) can be circumscribed by the physician with closed figures in the various planes, and therefore takes on the connotation of a volume defined and spatially positioned in known coordinates.

As said, the apparatus provides for a second support system for the at least one operative needle that, for example, carries a tissue-destructive radiation emission head, for example an optical fiber adapted to emit laser light. This second support system is configured to support the at least one needle during its movement, along a trajectory, for example defined by the support itself or undefined and left to the expertise of the surgeon, towards the area of the tissue to be destroyed. Advantageously, this second support system is in spatial relation with the first support system so that the position of the second support system, or at least of the area of support (i.e., of at least a part of the trajectory of the operative needle) of the operative needle (and hence said emission head) with respect to the ultrasonic probe, and with respect to the spatial volume investigated by the ultrasonic probe is known.

For example, the second support system defines guide trajectories the spatial development of which is known in the spatial reference system of the first support system in which the position of the ultrasonic probe is known. Therefore, the position of the second support system, namely the position of the trajectory, is virtually defined in the at least one first image, i.e., the positions that said at least one operative needle (and hence said respective emission head) can take in this first image are virtually known. This information is shown on the monitor and therefore the support system, or more precisely the virtual extension of the trajectory defined by the support system in the area to be treated, is displayed on the monitor of the surgeon, thereby allowing simulation of the treatment to be performed, for example the tissue-destructive treatment.

Preferably, definition of the area of intervention also provides for definition of a safety zone not to be invaded with the destruction treatment.

Preferably, the size of the volume that can be irradiated by the at least one emission head positioned virtually in the area of intervention is a function of one or more of the treatment parameters, including power of the emitted radiation, the amount of the emitted radiation, any particular movement of the head during the emission phase, etc.

For example, in the case of laser light, the size of the volume that can be irradiated by the at least one emission head positioned virtually in the area of intervention is a function of one or more of the treatment parameters: power of the radiation emitted by the radiation source, the amount or dose of energy emitted, number of pullback actions of the emission head from the treatment area, the length of the pullback actions.

A pullback action is a movement of the emission head, i.e., in the case of a needle with optical fiber, a reverse movement of the needle with the optical fiber from the treatment area (i.e., to extract the needle from the patient) with subsequent laser light emission as soon as the needle with the fiber is pulled back. This actions creates a particular form of the ablation volume in the treatment area that increases in the opposite direction to the insertion direction of the needles. The length of the pullback actions is meant as the distance, for example in mm, of reverse movement from the treatment area during pullback.

Obviously, the parameters to be set also include the number of emission heads (with the related parameters) inserted simultaneously in the area to be treated.

The heads can be arranged and all act together in the area to be treated or be inserted according to a given sequence (and act according to a given sequence). Obviously, also the positions of these heads contribute to forming the process parameters.

For example, the surgeon chooses the operating parameters and the position of the emission head and verifies whether the area of intervention is completely covered by the ablation volume emitted by the head. If this area is completely covered, without the ablation volume going beyond any safety zone or volume, i.e., without damaging anatomical structures that are to be left intact, then the surgeon decides that the simulation has been successful and can proceed with the actual treatment. Otherwise, the surgeon will change the aforesaid operating parameters including the position and/or the number of the emission heads used (one or more).

In preferred embodiments, the simulation module provides for real-time calculation of the simulation on the basis of the aforesaid parameters, i.e.: number of ablation heads, mutual positioning of the ablation heads, dose of energy applied by each ablation head, power of the ablation radiation source, possible presence of pullback actions, with definition of any pullback length/distance.

In preferred embodiments, the simulation module comprises a database of previous simulations performed on the basis of one or more of the aforesaid parameters, i.e.: number of ablation heads, mutual positioning of the ablation heads, dose of energy applied by each ablation head, power of the ablation radiation source, possible presence of pullback actions, with definition of any pullback length/distance; therefore, instead of implementing a live simulation, with calculation times correlated to the simulation, the surgeon can retrieve the results of a previously performed simulation with the same boundary conditions, given the same operating parameters indicated above, saving calculation time.

Advantageously, the electronic program can comprise a destructive treatment module of the area to be treated, which provides for the operation of setting one or more treatment parameters, corresponding to virtual treatment parameters set in the apparatus during operation of the simulation module; these parameters are, for example, power of the radiation emitted, the amount or dose of laser light energy emitted, number of pullback actions of the emission head from the treatment area, the length of the pullback actions.

According to preferred embodiments, the first support (and optionally guide) system for the ultrasonic probe comprises a support device, for example positioned on a trolley, which can comprise a slide or an assembly of slides, on which the ultrasonic probe can be applied. Therefore, it is possible to know, for example by means of encoder systems or similar, the position of the trolley, i.e., of the ultrasonic probe in the assembly of slides. Preferably, the support device can be fixed to a bed on which the patient is positioned, or in any case in a position fixed with respect to the bed with the patient. Preferably, the assembly of slides can provide one or more of the following degrees of freedom (knowing the position with respect to one or more of said degrees of freedom, in a reference system, for example, integral with the trolley/patient's bed): adjustment of the height from the ground; horizontal translation transverse to the edge of the bed with which the trolley/device is associated; adjustment of the inclination of the ultrasonic probe around a vertical axis and around a horizontal axis, to align the longitudinal axis of the ultrasonic probe, for example, with the axis of the rectum of the patient; adjustment of the distance of the end of the ultrasonic probe, for example from the anus of the patient, rotation of the probe around its longitudinal axis.

The support device of the ultrasonic probe, preferably the trolley, can comprise a constraint system, for example a cradle, which allows the probe to rotate around its longitudinal axis of development.

For example, the first support system for the probe can comprise a support device that comprises, for example, a jointed mechanical system, such as an articulated arm.

For example, this articulated arm or jointed mechanical system can be equipped at its end with a base on which a slide is slidingly arranged, on which a cradle that allows rotation of the probe around its principal axis is in turn provided; advantageously, for example, the slide allows the guided movement inside the natural orifice (for example the anus) of the patient along the principal axis and the cradle allows rotation of the probe inside the orifice around the principal axis.

In practice, the jointed mechanical system allows the probe to be positioned macroscopically in the operating area; a lock-release system of the jointed mechanical system is present, which makes it (and hence the probe) maneuverable when released and rigid when locked; for example, when locked only the fine positioning controls of the probe are available for further movements; for example, the fine positioning controls of the probe inside the orifice are those that allow the probe to move in longitudinal direction along the principal axis and to carry out rotations with respect to this axis.

Advantageously, once the support device is locked in position, the position of the probe and its orientation is preferably known with respect to the patient.

In preferred embodiments, the second support system that allows said at least one operative needle (and hence the related emission head, if present) to be guided along a given trajectory, in the, or close to the, area in which the intervention is to take place, for example the area with the tumor cells to be destroyed, is, for example, fixed to the support device that comprises the first support system for the ultrasonic probe. In this way the position of the second support system, and the development, i.e., the guide trajectory or trajectories for this head, is known in the reference system associated with this support device, i.e., a reference system in which the position of the ultrasonic probe is known.

Preferably, the second support system comprises a guide member that defines an array of guides, for example parallel to one another, such as through channels in which the operative needles can slide along parallel directions, distributed in an array, for example needles transporting emission heads, for example optical fibers, for laser light emission. These through channels define the guide trajectories of the operative needles, i.e., of the emission heads, and these trajectories are thus known with respect to a reference system that contains the guide member.

For example, this guide member with array of guides can have a fixed orientation with respect to the first support system of the ultrasonic probe, so that the spatial relation between the guides of the guide member and the position of the ultrasonic probe can be known. In other embodiments, this guide member can be associated with adjustment means of its orientation in space, with associated means adapted to identify the variation of orientation in space with respect to the first support system, and wherein these variations in orientation are transmitted to the apparatus, so that the position and relative orientation of the guide member (and hence of the guides/trajectories defined thereby), with respect to the first support system, i.e., with respect to the known position of the ultrasonic probe, are always known. The variation of the orientation of the guide member of the operative needles allows, for example, the operator to vary their possible position in the treatment area in the case in which a given orientation of these guides does not allow optimal treatment.

According to another aspect, the invention relates to a method of processing diagnostic images of a body volume.

For example, more in particular, this method of processing diagnostic images of a body volume acquired by means of an ultrasonic probe allows simulation of the positioning of operative needles in an areas to be treated; the probe used by the method has a principal axis for acquiring scans at least in a scan plane transverse and/or parallel to said principal axis; in particular, at least a first image acquired by the probe is transverse, i.e., relates to a plane transverse to the axis of the probe; the method provides for
acquiring in real time the rotational positions of the probe around its principal axis,
displaying said at least one first transverse image on a monitor related to a scan along a scan plane associated with said principal axis,
rotating said first transverse image on the monitor in a manner concordant with an acquired rotary movement of said probe around said principal axis in such a way that the content of said first image is fixed on the monitor.

Preferably, said first transverse image is acquired by scanning a plane transverse to the principal axis of the probe, or this first transverse image is produced by the reconstruction of scans of the probe along a plane longitudinal to the principal axis of the probe.

Preferably, the method is adapted to also process at least one second image relating to a scan of said probe along at least one plane longitudinal to said principal axis, and wherein said second image relates to a plane longitudinal to the axis of the probe.

Preferably, the method provides for display of the volume that can be irradiated by at least one destructive radiation virtual emission head positioned virtually in said area to be treated.

Preferably, the method provides that a form of circumscription of a safety zone surrounding the area of intervention is visible on said at least one first image.

Preferably, the method provides for
associating a virtual positioning structure for at least one operative needle carrying a virtual radiation emission head for the destruction of tissue, for example tumor cells, with said at least one first image,
displaying said virtual positioning structure of the needle with virtual emission head on said at least one first image
a form of circumscription of the area of intervention where tissue-destructive radiation is to be emitted being visible in said at least one first image
virtually positioning said at least one needle with virtual emission head on said virtual positioning structure so as to virtually emit the destructive radiation in said area of intervention,
verifying that the volume that can be irradiated by said at least one virtual emission head covers the entire area of intervention.

### Brief description of the drawings

The invention will now be better understood by following the description and the accompanying drawings, which illustrate a non-limiting example of embodiment of the invention. More in particular, in the drawing:

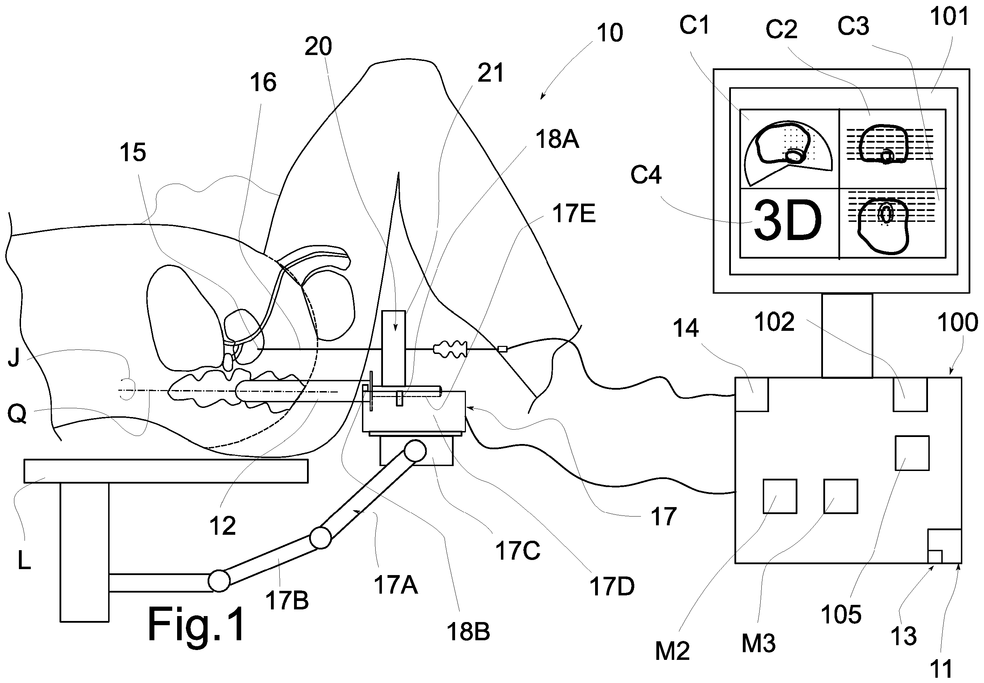

Fig. 1 represents a schematic view of the apparatus according to the invention, during a treatment in an area of the prostate, on a patient shown in a median or sagittal plane;
Fig. 2 represents an example of an ultrasonic probe that can be used in the apparatus according to the invention;
Fig. 3 represents a portion of guide needle of an optical fiber that acts as laser light emission head, to be used in the apparatus according to the invention;
Fig 4A represents a support and guide member for a guide needle of an optical fiber that acts as laser light emission head, to be used in the apparatus according to the invention;
Fig 4B represents a schematic view of a variant of guide member with respect to that of Fig. 4A;
Fig. 5 represents a diagram of the ultrasonic images on a monitor in the apparatus according to the invention, respectively a first image along a plane transverse to the axis of the ultrasonic probe used for the scan, a second image along a plane longitudinal to the axis of the probe, a third image relating to a view reconstructed by means of the information of the other two planes, and a fourth image (not shown in detail but represented by the reference "3D") relating to a three-dimensional reconstruction produced from the information of the other images;
Fig. 6 represents the diagram of Fig. 5 with the presence of an operative needle and the definition of an ablation area;
Fig. 7 represents a diagram relating to the first image on the monitor of the apparatus according to the invention, in which different ablation actions within the area to be treated are visible;
Fig. 8 represents a diagram relating to the first image on the monitor of the apparatus according to the invention, wherein the rotation of a point of the image in relation to the rotation of the probe around its principal axis is highlighted;
Fig. 9 represents the definition of the transverse PT, median/sagittal PM and frontal/coronal PF planes, relating to possible two-dimensional diagnostic imaging planes;
Fig. 10 represents a diagram of the steps of a method according to the invention, for example applied to the apparatus of the preceding figures.

### Detailed description of embodiments

With reference to the aforesaid figures, a medical apparatus using an ultrasound approach for natural orifices according to the invention is indicated as a whole with the number 10. More in particular, this example relates to a tissue-destructive radiation emission medical apparatus, for the treatment of benign and malignant diseases in the prostate or of the vaginal region. The specific example described relates to an ablation treatment of neoplastic tissue of the prostate.

This apparatus 10 comprises an ultrasound scanner 11 equipped with an ultrasonic probe 12, such as a preferably transrectal or endorectal endocavitary probe, capable of acquiring images relating to a spatial volume in the area surrounding the prostate. This ultrasonic probe 12 is of the type adapted to acquire the spatial volume by means of two movements, a first axial movement in which the probe is moved along a principal axis Q thereof and a second movement of rotation J around this principal axis Q, said probe being adapted to display said spatial volume in real time in at least one image on a monitor.

The apparatus further comprises a laser emission device 13 which provides a laser light source 14 operatively connected to one or more laser light emission heads 15, light that acts as tissue-destructive radiation, for example benign or malignant neoplastic cells, i.e., capable of performing ablation of the neoplastic area.

For example, an emission head 15 is the end of an optical fiber 15A operatively connected with the laser light source 14, and arranged in a guide needle 16, which can in turn be positioned in the treatment area, as better explained below.

The apparatus 10 comprises an electronic device for its management, indicated as a whole with 100.

It should be noted that, in another embodiment, the apparatus may also not include the ultrasound scanner 11, but means for interfacing therewith. For example, the electronic device 100 can comprise a video channel interfaced with an ultrasound scanner external to the apparatus, and in which the video channel acquires a stream of ultrasound images from the ultrasound scanner, and digitalizes them in the apparatus, for example by means of a frame grabber device. In this case, the image depth (which defines the aspect ratio) can be input by the user or the electronic device is equipped with numerical recognition that finds and interprets the depth numbers usually present on the screen of the ultrasound scanner.

Returning to the endocavitary ultrasonic probe 12, this is associated with a first support system 17, configured to support this probe in the area to be investigated. This first support system 17 is such that the position of the probe in the support system is known and, more generally, the position of the ultrasonic volume generated (i.e. the spatial volume investigated in the patient) in a reference system associated with the first support system is known, so that by moving the probe with respect to the support, the new position of the probe and of the ultrasonic volume generated is known. In this example, this first support system 17 also provides a guide system for the probe 12.

For example, the first support (and guide) system 17 for the endocavitary probe 12 comprises a support device 17A comprising, for example, a jointed mechanical system (for example an articulated arm) 17B that can be equipped at its end with a base 17C arranged sliding on which is a slide 17D provided with a cradle 17E that allows rotation of the probe 12 around the axis Q. The slide 17D allows the guided movement inside the rectum of the patient along Q, the cradle 17E allows rotation of the probe 12 inside the rectum of the patient around Q.

Rotational position indicators 18A are present around the principal axis Q and axial position indicators 18B are present along this principal axis Q, such as encoder systems or similar, for example in the case of manual movements of the slide/probe, or systems for measuring the movements of the actuator members are present in the case of actuators that automatically move the probe/slide. Therefore, it is possible to know the position of the slide, i.e., of the endocavitary probe in the assembly of slides.

The support device 17A can be fixed to the bed L on which the patient is positioned, or in any case in a fixed position with respect to the bed with the patient.

In practice, the probe is supported by a jointed mechanical system connected to the operating table, to the structure of the machine or to a stand on the ground. This jointed mechanical system can be composed of an articulated arm and a movement system that allows the probe to be positioned macroscopically in the operating area. The articulated arm has a lock/release system that makes it maneuverable when released and rigid when locked. When locked only the fine positioning controls are available for further movements. These fine positioning controls of the probe 12 inside the rectum are those produced by the slide 17D and by the cradle 17E, i.e., which allow the probe to move in longitudinal direction and to carry out rotations with respect to its axis.

Once the support device 17A is locked in place, the position of the probe and its orientation is known with respect to the patient.

The base 17C or the slide 17D can have a spirit level or inclination indicator that must allow perfect alignment with the operating table and hence the patient. Therefore, the operator acts on the positioning of the articulated arm so that before it is locked, the cradle and consequently the probe are levelled. This ensures a lower risk of injury for the patient and also minor deformation of the member, as when the patient is in gynecological position the prostate is aligned with the rectum, which is distended horizontally.

The endocavitary ultrasonic probe 12 is a volumetric probe, i.e., capable of acquiring images of an ultrasonic volume generated by the probe. The volume can be generated by the probe held stationary in the rectum, or by a movement of the probe in the rectum, according to the type of probe used.

For example, in this embodiment, the ultrasonic probe used is one of those described in the international patent application WO2020/212893.

For example, the ultrasonic probe 12 comprises an oblong body 12A, with a convex curved outer surface, extending along a longitudinal development along the axis Q of this body, wherein the body is provided with a plurality of ultrasonic sensors 12B facing out on the curved surface for emitting and receiving ultrasonic waves according to a combination of at least one rectilinear curtain, parallel to the axis of the probe and at least one curvilinear curtain, lying on a circumference in a plane orthogonal to the axis of the probe.

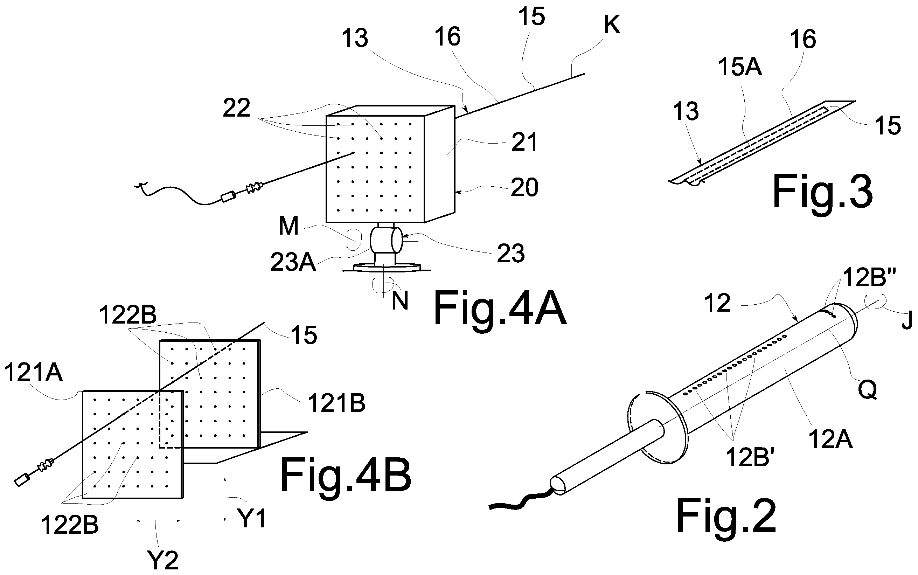

Fig. 2 shows the case of an ultrasonic probe with a single rectilinear curtain 12B', parallel to the axis of the probe and a single curvilinear curtain 12B'', lying on a circumference in a plane orthogonal to the axis of the probe.

Obviously, it is possible to use other types of transrectal/endorectal ultrasonic probes capable of scanning ultrasonic volumes in the body of the patient, through insertion into the rectum of the patient. For example, other probes in the form of oblong bodies can have ultrasonic sensors at the top of the end of the body, more or less in line with the body.

The probes indicated require a relative movement with respect to the area to be investigated, as the ultrasonic volume is generated by the combination of the front of the sensors with the movement of the probe. In other examples, it is possible to use ultrasonic endocavitary probes in which the body that contains the ultrasonic sensors remains stationary with respect to the area to be investigated, while it is the sensors that move on the body, in order to generate the ultrasonic volume.

The apparatus 10 further comprises a second support system 20, configured to support and guide at least one guide needle 16 carrying a related laser light emission head 15 (or several needles carrying relative heads 15), along a given trajectory K (or several trajectories K), in, or close to, the area with the neoplastic tissue to be destroyed.

For example, the second support system 20 comprises a support and guide member 21 that defines an array of guides, for example parallel to one another, such as through channels 22, in which the needles 16 transporting the optical fibers the ends of which form the emission heads can slide along parallel directions, distributed in an array. These through channels 22 define the guide trajectories K of the needles 16, and hence of the emission heads 15 contained therein.

This second support system 20 is in spatial relation with the first support system 17 of the endocavitary ultrasonic probe, so that the position of the trajectories K defined by the guide member 21 is known in a reference system in which the position of the endocavitary probe 12 is also known (i.e., the spatial volume investigated by said endocavitary probe is known).

For example, the guide member 21 is constrained to the support device 17A in a known way and oriented so that the through channels 22 for guiding the needles 16 are oriented towards the patient's perineum, so that the projection of the array of guides covers the area of the prostate, such o that a movement of the needles inside the perineum carries them, and hence the laser light emission heads, into the treatment area.

The support and guide member 21 can also allow insertion of needles oblique with respect to the longitudinal direction (i.e., oblique with respect to the axis Q) to treat portions of tissue whose access is obstructed by critical structures (bone or non perforable structures).

Therefore, for example, the support and guide member 21 is associated with a device 23 for adjusting the orientation in space of the guides 22, with associated one or more systems 23A (for example encoder systems) adapted to identify the variation of orientation in space with respect to the first support system to which it is fixed, and wherein these variations in orientation are transmitted to the apparatus 100, so that the position and relative orientation of the guide member 21 (and hence of the guides/trajectories K defined thereby), with respect to the support device 17A, are known at all times.

For example, this device 23 for adjusting the orientation in space of the guides 22 is provided with a hinge for constraint to the support device 17A, with a device for locking the member 21 in the desired position with respect to the support device. Advantageously, the encoder system 23A associated with the rotation of the guide member around the hinge axis (or axes) allows identification of the variation of orientation in space of the guide member, i.e., of the trajectories K associated therewith. The constraint hinge can adjust the orientation of the guide member also around several axes, for example two axes N and M (or three axes) orthogonal with one another.

Another example of device for adjusting the orientation in space of the guides 22 is provided in the example of Fig. 4B; in this case, the guide member comprises at least two arrays 121A, 121B with through guide channels 122A-122B arranged on two facing surfaces, with the arrays of the channels that are not rigidly aligned but have at least two degrees of freedom Y1, Y2; in particular, the proximal array 121A can move up/down and left/right while the distal array 121B remains stationary on the perineum, so that a needle changes inclination while its height in the array 121B remains unvaried and is varied in the array 121A. For example, the through channels 122A and 122B have an opening of larger area than the area of the section of the needles that are guided inside them. The relative movements can be obtained with vernier scales and encoders that transfer the amount of the movement to the system. In this way known oblique trajectories can be generated.

Further, the differentiated inclination can be performed by a set (not shown in the figures) of removable guide members, each defining an array of guides with different guide angles (each member is provided with parallel guides, while different members have different guide angles to the other members). In this case, the device for adjusting the orientation in space of the guides of the needles consists of a system for connecting the guide member to a support and a set of different guide members. In practice, as will be more apparent below, after the planning phase in which the directions of entry of the heads in the area to be treated is established, the guide members provided with a respective fixed angle coherent with the angle of the direction of entry defined by planning are mounted on the first support system.

Further, in another example not shown in the figures, the second support system 20 can only support the heads 13 without guiding them along a trajectory. For example, a support device can be present that supports the needles 16 without obligatorily directing them in one direction, in the manner of a fork or pivot. For example, this support device can be represented only by the array 121A (i.e., the array 122B is not present). The support position of the needle (i.e., of the fork/pivot) is known in the reference system, so that on the monitor, as will be better explained below, it is possible to virtually display a trajectory that develops from the support area to the area for positioning the head in the area to be treated. The surgeon is in any case free to move the head towards the treatment area, orienting it appropriately, handling the needle on the opposite side to the head with respect to the pivot point/fork. With a positioning system of the needles of this type, which has little or no guiding power (not capable of directing the needle in a specific direction) the physician is able, after having defined the point of entry of the needle in the perineum, to direct it as desired in order to reach the target tissue. As said, the support device has little guiding power and this allows the physician to direct the needle as desired in the target area, at the same time maintaining the feature of fixing the spatial entry coordinates with respect to the patient or to the first support system.

In other examples, not shown in the figures, the second support system can comprise a support, or support and guide, member for at least one operative needle, wherein this support member is rigidly constrained to the body of the probe, thereby moving with the probe when this is moved by the physician (the position of the probe and its orientation being known, the position and orientation of the support member of the needles is also known). There is a device for constraint/release of the support member with respect to the probe, when it is necessary to release them.

The electronic device 100 for managing the apparatus, comprising a monitor 101 (or other viewing system) available to the surgeon, and an electronic program 102 comprising different operating modules, including a tissue-destruction simulation module M2, better described below, and a module M3 relating to the actual intervention to destroy the tumor cells based on the simulations of the module M2 (in practice the module M2 forms a planning module of the subsequent intervention).

From an operative point of view, the use of the apparatus can take place, for example, as follows.

The patient is arranged on the bed L in prone position, for example with their knees bent and legs spread apart in gynecological position. The patient can be given a local anesthetic, for example a blocking anesthetic administered through the same guide needle used to guide a laser emission head.

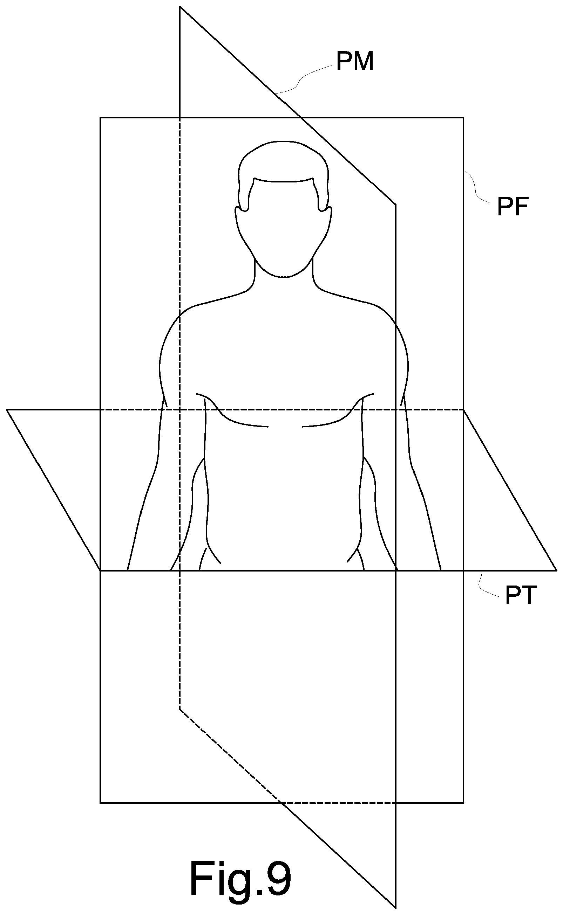

The endocavitary ultrasonic probe 12, by means of positioning of the articulated arm, is positioned inside the patient's anus, with the axis Q of the probe possibly aligned with the frontal/coronal plane PF and the median plane PM of the patient (see Fig. 9), or in any case roughly parallel to these two planes. The position of the probe is known.

From here the probe is moved longitudinally, following its axis Q inside the patient's anus and scans of a plurality of transverse planes are thus performed, thereby obtaining a first volumetric scan of the area to be treated, which comprises the patient's prostate. Likewise, by rotating the probe around the axis Q, scans can be taken of a bundle of longitudinal planes, which combined with the scans of the transverse planes, allow the quality of the volumetric scan of the area to be treated to be increased.

At least two flat images are, for example, visible on the monitor, a first image C1 relating to a transverse scan plane and a second image C2 relating to the longitudinal plane. In real time it is possible to see the scan of only one of the two planes, i.e., that of the set of active sensors, in this example the rectilinear curtain 12B', parallel to the axis of the probe or the curvilinear curtain 12B'', lying on a circumference in a plane orthogonal to the axis Q of the probe. For example, in the image C1 (transverse view) a segment PL is visible, which indicates the longitudinal plane relating to the scan visible in the image C2.

By combining the two transverse and longitudinal planes, it is possible to reconstruct a third image C3 relating to the frontal/coronal plane. This third image can also be shown on the monitor. A fourth image C4 showing a three-dimensional image of the scanned zone formed by the combination of the three planes can also be shown on the monitor.

As said, in real time it is possible to see the scan on only one of the two planes. Therefore, when the real time display relates to the transverse plane, an ultrasonic plane reconstructed from the previous acquisition of volume in the longitudinal plane will be seen in the longitudinal plane (chosen from a plurality of longitudinal ultrasonic planes acquired previously). Vice versa, if the real time display is in the longitudinal plane, then an ultrasonic plane reconstructed in the image of the transverse plane (chosen from a plurality of ultrasonic transverse planes acquired previously) will be seen.

The images on the monitor show the prostate G and other anatomical structures of the patient, for example the urethra (not shown in the figures). The position (i.e. its spatial coordinates) of the volume referred by these three images are known, as the position of the ultrasonic probe 12 in a given reference system associated with the first support system 17A is known.

After performing the three-dimensional scan, it is possible to activate the planning/simulation module M2.

The surgeon must decide how much of the prostate to subject to ablation through the laser radiation produced by the heads 15, taking care not to damage anatomical structures that are to be protected.

To do this, the electronic program 101 makes it possible to highlight an area around the neoplastic area T of the prostate to be treated, identified with H1 in the first image, with H2 in the second image of the longitudinal plane and H3 in the coronal plane, which defines a margin of destructive intervention by the laser emitted by the ablation heads 15 larger than the tumor itself, in order to guarantee complete destruction of all the neoplastic cells. For example, this area, also called ablation margin, is defined by a volume spaced from the contour H1-H3 of the neoplasia, for example by a given distance, for example from 2 to 5 mm, according to the surgeon's indications. In practical terms this area of intervention is, in each image C1-C3, a closed form V1-V3 that includes the neoplastic area T, for example a figure that follows the form of the tumor and spaced from this by a given distance (in practice a figure offset with respect to the contour of the tumoral area T). In other examples, the form of the margin of intervention can be delimited differently, for example by means of a well-defined geometric figure. The ablation must involve all of the area inside the volumetric form V1-V3.

Moreover, the electronic program 102 allows a safety margin S1-S3 to be defined, i.e., a form that surrounds (in each of the three flat images C1-C3, or in the equivalent three-dimensional image) the margin of intervention V1-V3 in order to define a safety zone outside which no ablation must be performed, to guarantee the anatomical structures surrounding the tumor.

Also in this case, this safety margin S1-S3 can be defined by an offset of the tumor area, or by a well-defined form. In the accompanying figures, this safety margin S1-S3 is represented, for example, by an ellipsoidal volume.

The electronic program 102 also displays in the first, second and third images C1-C3 the trajectories K for the ablation heads 15. In fact, as said, the position of the trajectories K is known with respect to the ultrasonic volume detected by the probe 12 (the position of the probe 12 and hence of the ultrasonic volume detected thereby, and the orientation of the guide member 21 are known in a same reference system). In a first example, the trajectories K are rectilinear and orthogonal to the transverse plane, so that in the image C1 these trajectories are represented, for simplicity, by points or areas of points arranged in an array, while in the images C2 and C3 by parallel straight lines (indicated by dashed lines).

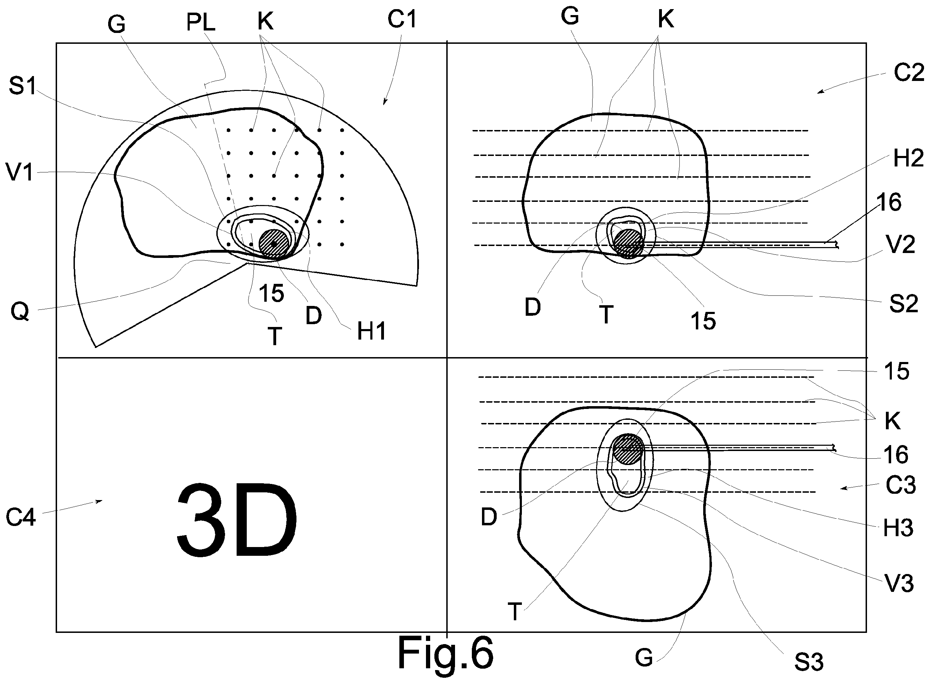

It is thus possible to virtually position the laser emission heads 15 around or inside the area of intervention defined by the form V1-V3 For example, Fig. 6 schematizes a needle 16 carrying the optical fiber with the emission head 15 at the end thereof.

By means of the simulation/planning module M2 of the electronic program, the surgeon virtually positions one or more heads in the areas considered most suitable to perform ablation and virtually sets the treatment parameters that will determine ablation.

These parameters provide for indication of the power of the laser light emitted by the laser light source (for each emission head or for all the heads), the amount or dose of energy of laser light emitted by each head, possible number of pullback actions of the emission head from the treatment area towards the outside and the length of these pullback actions.

These parameters define a volume that can be irradiated (ablation volume or area) D with laser light, or ablation volume, for each image C1, C2, C3 that is displayed on the monitor. In this example, this ablation volume is schematized by means of a spherical volume, and hence, in the various images C1-C3, by circles. The form of the ablation volume can differ from that indicated, and be a function, for example, also of any pullback actions set.

The heads can be arranged and all act together in the area to be treated or be inserted in a given sequence (and act in a given sequence). Obviously, also the number and the positions of these heads contribute to forming the parameters of the ablation process.

By viewing the ablation volume in the various images, the surgeon is therefore able to understand whether the setting of the treatment parameters is sufficient to perform an ablation of the whole of the treatment area V1-V3 without going beyond the safety zone S1-S3.

If not, the surgeon changes the treatment parameters, for example changing position and/or number of the emission heads, laser power, etc. and performs a new simulation.

The simulation of the effect of one or more ablation heads 15 in the area to be treated can take place in different ways.

A first method provides for an "in-line" simulation, i.e., the surgeon sets the parameters indicated above and launches a simulation with these parameters. The electronic program calculates directly, by means of suitable algorithms (described in more detail below), the ablation area D and displays it on the monitor.

To speed up the intervention times without waiting for the calculation times of the electronic program, a second method provides for the use of a database of previous simulations. In practice, the results of simulations for every possible combination of the input parameters specified above are stored in this database, given the specific equipment used (i.e., the type of ablation heads, the number of guides and their mutual positioning), i.e.:
- number of optical fibers to carry the ablation laser light
- mutual positioning of the ends of these optical fibers (needles carrying the optical fibers) that define the ablation heads
- dose of energy applied by each ablation head
- power of the laser source
- pullback actions
- pullback length/distance

In practice, a computer has calculated a very high number of simulations for a very large number of combinations, which in substance comprises all the possible cases of ablation with given laser sources, given ablation heads and given guide members for the needles with the optical fibers. The surgeon, after having positioned the ablation heads 15 on the monitor along the virtual trajectories K, and assuming all the values of the parameters cited above, merely requires to call up the desired simulation from the database for the result to be displayed immediately, without waiting for the calculation times of the simulation.

The simulations are obtained using a mathematical model based on the modified Pennes' equation: $cρ \frac{\partial T}{\partial t} = ∇ ⋅ \left(k∇\left(T\right)\right) + {Q}_{laser} + {Q}_{perf} + {Q}_{e} + {Q}_{met}$
where T is the temperature of the tissue in Kelvin, ρ is the density of the tissue [kg / cm3], c is the specific heat of the tissue [J·kg⁻¹·K⁻¹], κ is the thermal conductivity of the tissue [W·m⁻¹·K⁻¹]. Qlaser is the energy added externally per unit of volume, Qperf is the heat transfer deriving from blood perfusion, Qe is the heat exchange due to vaporization (boiling) of the water and Qmet is the metabolic heat exchange (which is negligible in laser ablation and removed from the calculations).

The optical field can be calculated through the diffusion equation that is applied to the turbid media where the scattering coefficient is not negligible as is tissue or through modelling of the Gaussian field output from an optical fiber with a flat tip. If the emitter is of complex type, for example side firing or ring firing, the spatial optical field must be remodeled time by time, taking into account the principle of energy conservation in the vacuum.

The optical distribution that is output from the optical fiber is calculated at each instant in time, through the Pennes' equation the thermal field that acts through the Arrhenius equation on tissue denaturation is obtained. The tissues denatured by heat change optical and also thermal properties, as well as changed blood perfusion due to coagulation of the tissues. This simulation pattern takes account of the changes caused by coagulation updating the optical and thermal parameters of the portions of tissue affected by denaturation to then start a new cycle for a subsequent instant of time.

The Arrhenius damage model is the following: $Ω = - ln \left[\frac{c \left(t\right)}{c \left({t}_{0}\right)}\right] = {\int }_{0}^{t} \frac{k}{ℏ} T ⋅ exp \left(\frac{Δ S}{R}\right) ⋅ exp \left(-\frac{Δ H}{RT}\right) dt ′$
where c(t₀) is the initial concentration of the cells and c(t) is the concentration of vital cells at the instant t. Δ*S* : activation entropy [cal mol⁻¹ K⁻¹], Δ*H* : activation enthalpy [kcal mol⁻¹], *R* : gas constant, T: temperature.

When Ω varies the following percentage values of denatured tissue are obtained with respect to the initial condition (native tissue):
Ω=3 - 95% of denatured tissue;
Ω=2 - 86% of denatured tissue;
Ω=1 - 63% of denatured tissue;
Ω=0.7 - 50% of denatured tissue;
Ω=0.6 - 46% of denatured tissue;

Depending on whether a reference value of Ω is chosen for the coagulated tissue, for example Ω=1, the entire time simulation of a volume will give rise to a set of closed surfaces delimiting the denatured part from the undenatured part.

Therefore, it is clear that the various parameters listed above on which the simulations are based must also include the selected value of Arrhenius damage Ω.

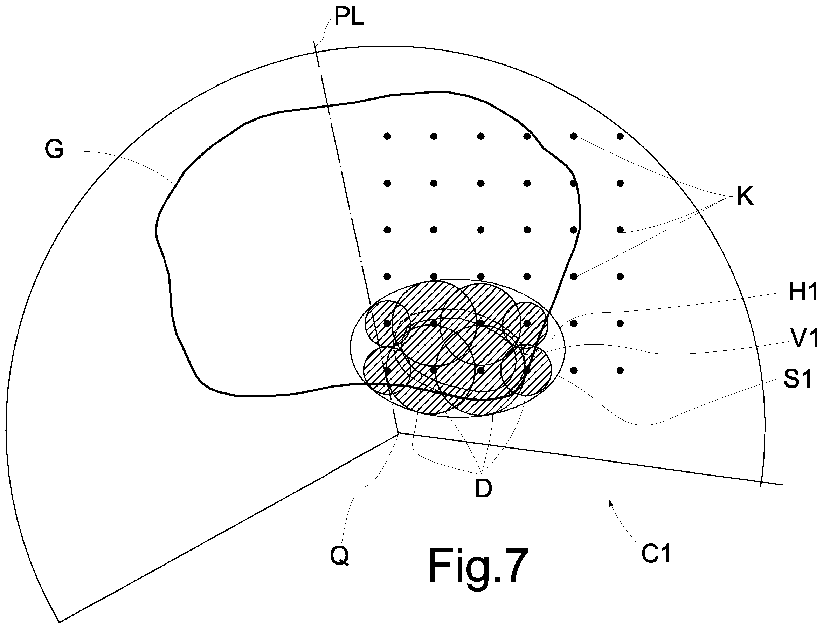

When the result of the simulation (live, or taken from the database) is positive, i.e., shows the treatment area V1-V3 completely superimposed on the ablation areas of the ablation heads, for example as in the case of Fig. 7 (naturally, an analogous verification must also be carried out in the other two images C2 and C3), the surgeon uses the parameters that gave the positive result in the simulation module M2 and performs the actual treatment, inserting the needles 16 with the heads 15 in the desired positions through the perineum and supplying the laser with the desired power, and operating the desired movements of the needles, following the indications of the simulation.

Advantageously, the electronic device 100 for managing the apparatus comprises a correction module 105 of the first image relating to the transverse plane, visible on the monitor. This correction module allows the first image C1 to be rotated on the monitor in a manner concordant with a rotary movement of the probe 12 around the principal axis Q, so that the content of the first image, which advantageously will relate to the area to be treated, will be fixed on the monitor during rotation of the probe.

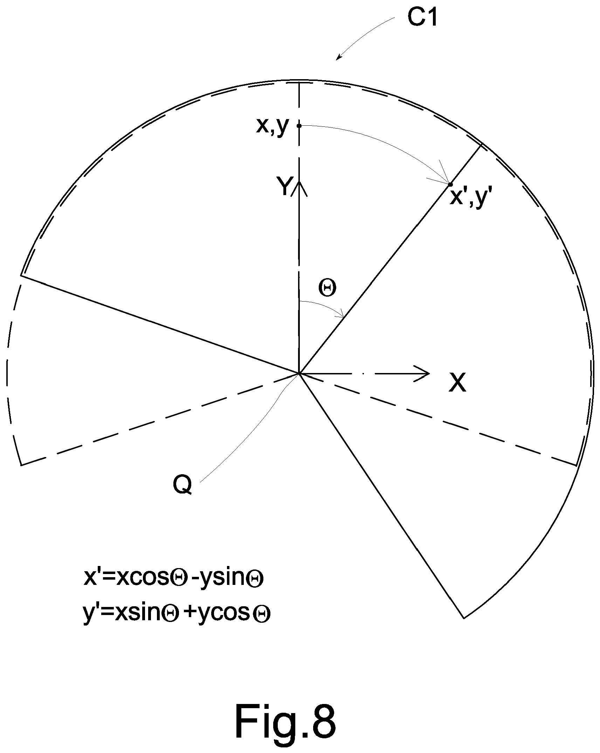

For example, Fig. 8 shows the correction mode set by the correction module 105. For example, the encoder 18A verifies by what angle θ the probe 12 is rotated around its principal axis Q. This angle is used for the angular correction of each point of the image C1. For example, considering each point or pixel of the first image on the monitor, given the angular coordinate of the point x and y, on a Cartesian reference system whose origin coincides with the axis Q, the coordinate correction on the abscissa will be x ' = x cos θ - y sin θ and the correction on the ordinate will be y ' = x sin θ + y cos θ.

The correction module 105 can be activated, for example, after initial acquisition of the volume of the area to be treated, for example during the planning step or during the actual treatment step.

This correction will allow the surgeon, when moving the probe around the axis Q, for example to try to visualize anatomical structures or trajectories or needles that, for example, are clearly visible in the first image relating to the transverse plane, while, for example, they are not clearly visible in the second image relating to the longitudinal planes. The surgeon rotates the probe and searches for what they are looking for on the second image, while the content of the first image relating to the patient's anatomical structures, i.e., everything that is fixed with respect to the patient's bed, remains substantially stationary, making it easier to visualize everything. On the contrary, if the first image were not to rotate, as is normally the case in ultrasound systems with probes of this type, the content of the first image would rotate with the probe, with the risk of creating confusion for the surgeon. The invariance of the patient's anatomical structures during rotation of the probe makes the planning, and optionally the treatment, process more intuitive, reducing the risks of error.

In cases in which it is necessary to have trajectories of the needles that are not parallel to the axis of the probe, for example because the structures external to the organ could prevent a "parallel trajectory", it is advantageous to have all three images C1, C2 and C3, one for each reference plane (transverse, longitudinal and coronal), and preferably also the 3D reconstruction (image C4) so as to clearly see the treatment area. In this way, the coordinates of the tip of the needle 16 are defined in space through the three transverse, longitudinal and coronal planes and the projection on these and the trajectories are, for example, defined by the coordinates XY of the points of the array 21 (or 121A) that fix the points of entry in the patient.

The probe 12 generates ultrasonic images of planes orthogonal to one another and therefore cannot, in the case of curved trajectories, show all the route/trajectory of the needle but only a portion given by the intersection of the trajectories with the plane scanned, i.e., in practice points or small sections. This is also the case for the ablation figures determined by the coordinates of the tips of the needles and by their inclination that is "cut" and reconstructed and shown according to the intersection of the ultrasonic scan plane with their spatial position. Advantageously, in this case, planning works on volumes reconstructed from previous acquisition. Instead, in the 3D reconstruction of the image C4, the trajectories in space, the area of intervention, the critical structures and the organ boundaries can be visible and the physician is therefore able to reach the treatment area by moving both the cursors (which simulate the tip of the needle) and the point of penetration on the perineum defined by the grid.

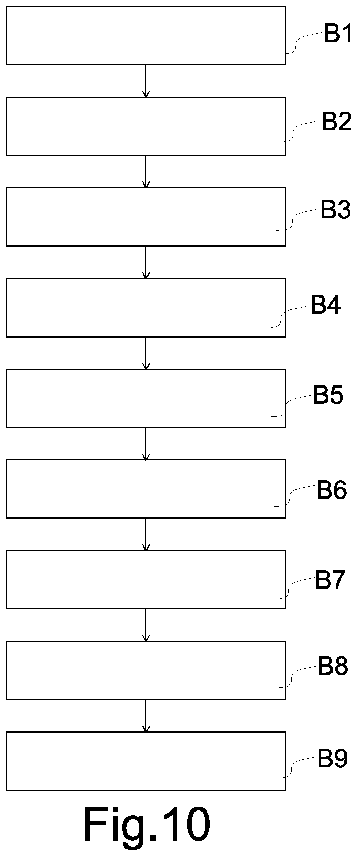

Fig. 10 indicates a block diagram of the method of processing diagnostic images of a body volume described below.

In particular, the method provides for a first block B1 relating to the real time acquisition of rotational positions of the probe 12 around the principal axis Q.

The method also provides for a block B2 relating to the real time acquisition of axial positions of the probe 12 along the principal axis Q.

The method further provides for a block B3 relating to the display of at least one first image C1 and at least one second image C2 on a monitor 101, the first image relates to an image transverse to the principal axis Q and the second image is an image longitudinal to the axis Q.

For example, the at least one first image C1 relates to scans in a plane transverse to the principal axis Q and the at least one second image relates to a scan with respect to a plane longitudinal to the axis Q.

A block B4 relates to the rotation of the first transverse image C1 on the monitor in a manner concordant with the acquired rotary movement of the probe around the principal axis in such a way that the content of the first image is fixed on the monitor.

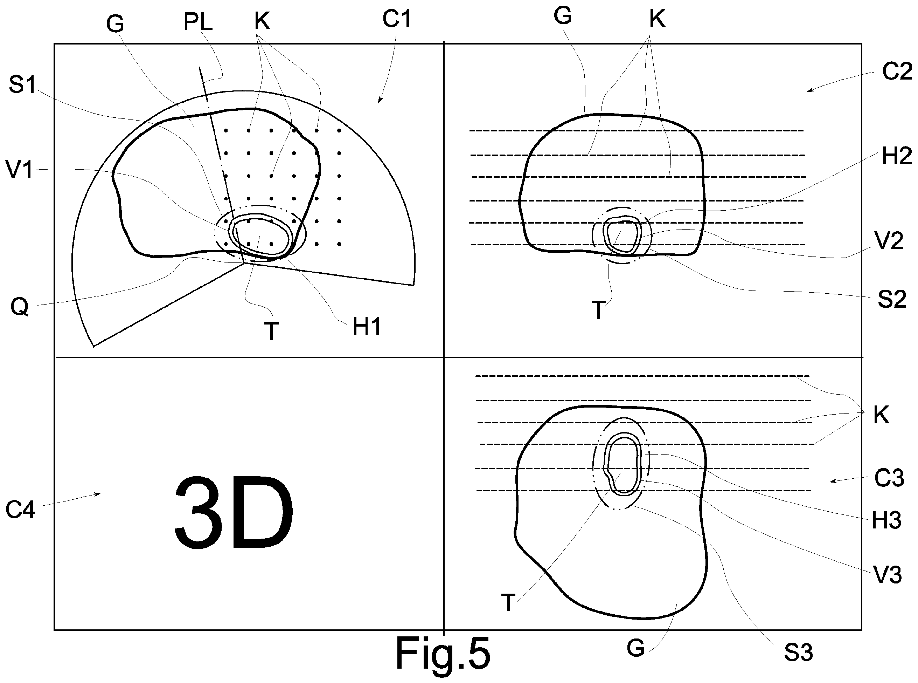

A subsequent block B5 provides for definition of the area of intervention V1-V3 in which to emit the laser light for ablation of the tumor cells on the images, as shown in Fig. 5.

An optional block B6 also provides for definition of a safety zone p S1-S3 that circumscribes the area of intervention on the images, and this circumscription is also visible on the images C1-C3, area beyond which the damage to the tissues caused by the ablation heads must not pass.

A subsequent block B7 relates to the virtual positioning, on the images C1-C3, of one or more virtual emission heads at the virtual positioning structure so as to virtually emit the destructive radiation in the area of intervention V1-V3, as shown in Fig. 6.

An optional block B8 relates to the display of the volume (volumes) D that can be irradiated by the virtual emission head positioned virtually in the area of intervention V1.

A block B9 relates to verification that the volume D that can be irradiated by the virtual emission head (heads) covers the whole of the area of intervention V1-V3 and that it does not go beyond the safety zone S1-S3.

It is understood that the description illustrated only represents possible non-limiting embodiments of the invention, which can vary in forms and arrangements without departing from the scope of the concept underlying the invention. Any reference numbers in the appended claims are provided purely in order to facilitate the reading thereof in the light of the foregoing description and of the accompany drawings and do not in any way limit the scope of protection.

## Claims

1. Medical apparatus (10) using an ultrasound approach for natural orifices comprising
• an ultrasonic probe (12) adapted to be introduced into a natural orifice of a body, having a principal axis (Q), configured to acquire a spatial volume by acquiring scans along at least one scan plane associated with said principal axis (Q) plane;
• a vision system comprising a monitor (101) on which at least one first image (C1) relating to an image in a plane transverse (PT) to said principal axis (Q) is visible;
• a first support system (17), configured to support said ultrasonic probe (12) in the area to be investigated, wherein said support system (17) is adapted to constrain said probe (12) to allow two movements, respectively a first axial movement in which the probe (12) is moved along a principal axis (Q) thereof and a second movement of rotation around said principal axis (Q), the position of said probe (12) in said first support system (17) being known;
• position indicators (18A, 18B) for
rotational position around, and
position along,
said principal axis (Q) of said probe (12) in said first support system (17);
• at least one operative needle (16) adapted to be introduced into the body portion;
• a second support system (20), configured to support said at least one operative needle (16) and to allow its movement along a trajectory (K), towards the area of intervention in which it is adapted to operate, said second support system (20) being spatially related to said first support system (17) for which the support position of said operative needle (16) is known with respect to said ultrasonic probe (12), and with respect to the spatial volume investigated by said ultrasonic probe (12);
• an electronic device (100) for managing the apparatus (10),
**characterized in that** said electronic device (100) comprises a correction module of said at least one first image (C1) visible in the monitor (101), adapted to rotate said at least one first image (C1) on the monitor (101) in a manner concordant with the rotary movement of said probe (12) around said principal axis (Q), so that the content of said at least one first image (C1) relating to the area to be treated is fixed on the monitor (101) during rotation of said probe (12).

2. Apparatus (10) according to claim 1, wherein said scan plane is
• a plane transverse (PT) to said principal axis (Q) of the probe (12), and wherein said at least one first image (C1) relating to an image in a plane transverse (PT) to said principal axis (Q) comes from at least one scan of said probe (12) in said plane transverse (PT) to said principal axis (Q), or
• a plane longitudinal to said principal axis (Q), and wherein said at least one first image (C1) relating to an image in a plane transverse (PT) to said principal axis (Q) is reconstructed from a plurality of scans performed in a plane longitudinal to said principal axis (Q).

3. Apparatus (10) according to one or more of the preceding claims, wherein said monitor (101) contains at least one second image (C2) relating to a scan of said probe (12) in at least one plane longitudinal to said principal axis (Q).

4. Apparatus (10) according to one or more of the preceding claims, wherein said ultrasonic probe (12) comprises an oblong body (12A) having a convex curved outer surface extending along a longitudinal development of said body (12A), wherein said body (12A) is provided with a plurality of ultrasonic sensors (12B) facing out on the curved surface for emitting and receiving ultrasonic waves; preferably, said curved surface of said ultrasonic probe (12) is substantially cylindrical and has an axis parallel to the longitudinal development of said oblong body (12A); preferably, the ultrasonic sensors (12B) of the probe (12) are arranged according to at least one rectilinear curtain (12B') parallel to the axis (Q) of the probe (12), and at least one curvilinear curtain (12B") lying on a circumference in a plane orthogonal to the axis (Q) of the probe (12).

5. Apparatus (10) according to one or more of the preceding claims, wherein said ultrasonic probe (12) is an endocavitary ultrasonic probe (12), preferably of the transrectal type.

6. Apparatus (10) according to one or more of the preceding claims, wherein said first support system (17) is adapted to constrain said probe (12) to permit only said two movements: the first axial movement in which the probe (12) is moved along a principal axis (Q) thereof, and the second movement of rotation around said principal axis (Q).

7. Apparatus (10) according to one or more of the preceding claims, wherein said at least one operative needle (16) is a guide needle (16) movable along a trajectory (K) defined at least in part by said second support system (20); preferably at least one tissue-destructive radiation emission head (15) is arranged in said at least one guide needle (16), preferably said electronic device (100) for managing the apparatus (10) comprises an electronic program (102) having a tissue destruction simulation module (M2) which provides for the operations of spatial definition, on at least said first image (C1) of the area of intervention where the destructive treatment is to be performed, display of a simulation of the virtual position of at least one said emission head (15) in said area of intervention on said at least one first image (C1) shown on a monitor (101); preferably said electronic program (102) providing for the display on said at least one image (C1, C2, C3) on said monitor (101), of at least one virtual trajectory (K) developing from the support position for said at least one emission head (15) on said second support system (20), wherein said virtual trajectory (K) defines a path for said emission head (15) towards the virtual position in the area of intervention.

8. Apparatus (10) according to claim 7, wherein said electronic program (102) provides for the display, on said at least one first image (C1) shown on a monitor (101), of a simulation of the volume (D) that can be irradiated by said at least one emission head (15) positioned virtually in said area of intervention, and wherein the size of the volume (D) that can be irradiated by said at least one emission head (15) positioned virtually in said area of intervention is a function of one or more of the following parameters: power of the emitted radiation, the amount or dose of energy of the emitted radiation, any movement of the at least one emission head (15) during the emission phase, the length of said possible movement, the Arrhenius damage value.

9. Apparatus (10) according to claims 3 and 7, wherein said simulation operations are also performed on said second image (C2) relating to a scan of said probe (12) in at least one plane longitudinal to said principal axis (Q).

10. Apparatus (10) according to one or more of claims 7 to 9, wherein said electronic program (102) comprises a destructive treatment module (M3) of the area to be treated, said electronic program (102) providing for the operation of setting treatment parameters, corresponding to virtual treatment parameters set in the apparatus (10) during operation of said simulation module (M2), said parameters comprising one or more of the following: number of emission heads (15) positioned at the emission zone, position of the at least one emission head (15) in the emission zone, power of the radiation emitted by the at least one emission head (15), the amount or dose of energy of the radiation emitted by the at least one emission head (15), any movement of the at least one emission head (15) during the emission phase, the length of said possible movement.

11. Apparatus (10) according to one or more of claims 7 to 10, wherein said simulation module (M2) provides for
• real-time calculation of the simulation on the basis of one or more of the following parameters: number of ablation heads (15), mutual positioning of the ablation heads (15), dose of energy applied by each ablation head (15), power of the ablation radiation source (14), possible presence of pullback actions, with definition of any pullback length/distance, or
• a database of previous simulations performed on the basis of one or more of the following parameters: number of ablation heads (15), mutual positioning of the ablation heads (15), dose of energy applied by each ablation head (15), power of the ablation radiation source (14), possible presence of pullback actions, with definition of any pullback length/distance, so that the surgeon can retrieve the results of a previously performed simulation with the same operating parameters indicated above, saving calculation time.

12. Method of processing diagnostic images of a body volume, including at least a first image (C1) acquired by means of an ultrasonic probe (12), for simulating the positioning of operative needles (16) in an area to be treated, wherein said ultrasonic probe (12) has a principal axis (Q) for acquiring scans at least in a scan plane transverse (PT) and/or longitudinal to said principal axis (Q), and wherein said first image (C1) relates to a plane transverse to the axis (Q) of the probe (12), said method providing for
- acquiring in real time the rotational positions (18A) of said probe (12) around said principal axis (Q),
- displaying said at least one first transverse image (C1) on a monitor (101) related to a scan along a scan plane associated with said principal axis (Q),
- rotating said first transverse image (C1) on the monitor (101) in a manner concordant with an acquired rotary movement of said probe (12) around said principal axis (Q) in such a way that the content of said first image (C1) is fixed on the monitor (101).

13. Method of processing diagnostic images according to the preceding claim, adapted to further process at least a second image (C2) relating to a scan of said probe (12) in at least one plane longitudinal to said principal axis (Q), and wherein said second image (C2) relates to a plane longitudinal to the axis (Q) of the probe (12).

14. Method of processing diagnostic images according to one or more of the preceding claims, which provides for displaying the volume (D) that can be irradiated by said at least one virtual emission head (15) positioned virtually in said area of intervention.

15. Method of processing diagnostic images according to one or more of the preceding claims, wherein a form of circumscription of a safety zone (S1, S2, S3) surrounding the area of intervention is visible on said at least one first image (C1).

16. Method of processing diagnostic images according to one or more of the preceding claims, which provides for
- associating a virtual positioning structure for at least one virtual radiation emission head (15) for the destruction of tumor cells with said at least one first image (C1),
- displaying said virtual positioning structure of the virtual emission head (15) on said at least one first image (C1),
- a form of circumscription of the area of intervention (V1, V2, V3) where tissue-destructive radiation is to be emitted being visible in said at least one first image (C1),
- virtually positioning said virtual emission head (15) on said virtual positioning structure so as to virtually emit the destructive radiation in said area of intervention,
- verifying that the volume (D) that can be irradiated by said at least one virtual emission head (15) covers the entire area of intervention (V1, V2, V3).

## Patentansprüche

1. Medizinisches Gerät (10) zur Anwendung von Ultraschall an natürlichen Körperöffnungen, umfassend
- eine Ultraschallsonde (12), die dazu ausgebildet ist, in eine natürliche Körperöffnung eingeführt zu werden, mit einer Hauptachse (Q), die dazu eingerichtet ist, ein räumliches Volumen durch Erfassung von Scans entlang mindestens einer der Hauptachse (Q) zugeordneten Scanebene zu erfassen;
- ein bildgebendes System, umfassend einen Monitor (101), auf dem mindestens ein erstes Bild (C1) sichtbar ist, das sich auf eine Ebene (PT) quer zur Hauptachse (Q) bezieht;
- ein erstes Halterungssystem (17), das dazu eingerichtet ist, die Ultraschallsonde (12) im zu untersuchenden Bereich zu halten, wobei das Halterungssystem (17) dazu ausgebildet ist, die Sonde (12) so festzulegen, dass zwei Bewegungen möglich sind, nämlich eine erste axiale Bewegung, bei der die Sonde (12) entlang ihrer Hauptachse (Q) bewegt wird, und eine zweite Drehbewegung um die Hauptachse (Q), wobei die Position der Sonde (12) in dem ersten Halterungssystem (17) bekannt ist;
- Positionsanzeiger (18A, 18B) für die Drehposition um die Hauptachse (Q) sowie die Position entlang der Hauptachse (Q) der Sonde (12) in dem ersten Halterungssystem (17);
- mindestens eine Operationsnadel (16), die dazu ausgebildet ist, in den Körperabschnitt eingeführt zu werden;
- ein zweites Halterungssystem (20), das dazu eingerichtet ist, die mindestens eine Operationsnadel (16) zu halten und deren Bewegung entlang einer Bahn (K) in Richtung des Eingriffsbereichs, für den sie bestimmt ist, zu ermöglichen, wobei das zweite Halterungssystem (20) räumlich mit dem ersten Halterungssystem (17) in Beziehung steht, für das die Halteposition der Operationsnadel (16) in Bezug auf die Ultraschallsonde (12) bekannt ist, sowie in Bezug auf das von der Ultraschallsonde (12) untersuchte räumliche Volumen;
- eine elektronische Vorrichtung (100) zur Steuerung des Geräts (10),
**dadurch gekennzeichnet, dass** die elektronische Vorrichtung (100) ein Korrekturmodul für das mindestens eine erste Bild (C1) umfasst, das auf dem Monitor (101) sichtbar ist, das dazu ausgebildet ist, das mindestens eine erste Bild (C1) auf dem Monitor (101) in Übereinstimmung mit der Drehbewegung der Sonde (12) um die Hauptachse (Q) zu drehen, so dass der Inhalt des mindestens einen ersten Bildes (C1), der sich auf den zu behandelnden Bereich bezieht, während der Drehung der Sonde (12) auf dem Monitor (101) fixiert bleibt.

2. Gerät (10) nach Anspruch 1, wobei die Scanebene
- eine Ebene quer (PT) zur Hauptachse (Q) der Sonde (12) ist, und wobei das mindestens eine erste Bild (C1), das sich auf ein Bild in einer Ebene quer (PT) zur Hauptachse (Q) bezieht, aus mindestens einem Scan der Sonde (12) in der Ebene quer (PT) zur Hauptachse (Q) stammt, oder
- eine zur Hauptachse (Q) längs verlaufende Ebene ist, und wobei das mindestens eine erste Bild (C1), das sich auf ein Bild in einer zur Hauptachse (Q) quer verlaufenden Ebene (PT) bezieht, aus einer Vielzahl von Scans rekonstruiert wird, die in einer zur Hauptachse (Q) längs verlaufenden Ebene durchgeführt wurden.

3. Gerät (10) nach einem oder mehreren der vorstehenden Ansprüche, wobei der Monitor (101) mindestens ein zweites Bild (C2) enthält, das sich auf einen Scan der Sonde (12) in mindestens einer zur Hauptachse (Q) längs verlaufenden Ebene bezieht.

4. Gerät (10) nach einem oder mehreren der vorstehenden Ansprüche, wobei die Ultraschallsonde (12) einen länglichen Körper (12A) mit einer konvex gekrümmten Außenfläche aufweist, die sich entlang einer Längsausdehnung des Körpers (12A) erstreckt, wobei der Körper (12A) mit einer Vielzahl von Ultraschallsensoren versehen ist (12B), die auf der gekrümmten Oberfläche nach außen gerichtet sind, um Ultraschallwellen auszusenden und zu empfangen; wobei vorzugsweise die gekrümmte Oberfläche der Ultraschallsonde (12) im Wesentlichen zylindrisch ist und eine Achse aufweist, die parallel zur Längsausdehnung des länglichen Körpers (12A) verläuft; wobei vorzugsweise die Ultraschallsensoren (12B) der Sonde (12) entlang mindestens eines geradlinigen Vorhangs (12B') parallel zur Achse (Q) der Sonde (12) und entlang mindestens eines gekrümmten Vorhangs (12B") angeordnet sind, der auf einem Umfang in einer zur Achse (Q) der Sonde (12) orthogonalen Ebene liegt.

5. Gerät (10) nach einem oder mehreren der vorstehenden Ansprüche, wobei die Ultraschallsonde (12) eine endokavitäre Ultraschallsonde (12) ist, vorzugsweise vom transrektalen Typ.

6. Gerät (10) nach einem oder mehreren der vorstehenden Ansprüche, wobei das erste Halterungssystem (17) dazu ausgebildet ist, die Sonde (12) so festzulegen, dass nur die beiden Bewegungen zulässig sind: die erste axiale Bewegung, bei der die Sonde (12) entlang ihrer Hauptachse (Q) bewegt wird, und die zweite Drehbewegung um die Hauptachse (Q).

7. Gerät (10) nach einem oder mehreren der vorstehenden Ansprüche, wobei die mindestens eine Operationsnadel (16) eine Führungsnadel (16) ist, die entlang einer Bahn (K) bewegbar ist, die zumindest teilweise durch das zweite Halterungssystem (20) definiert ist; wobei vorzugsweise zumindest ein gewebezerstörender Strahlungsemissionskopf (15) in der zumindest einen Führungsnadel (16) angeordnet ist, wobei vorzugsweise die elektronische Vorrichtung (100) zur Steuerung des Geräts (10) ein elektronisches Programm (102) umfasst, das ein Modul (M2) zur Simulation der Gewebezerstörung aufweist, welches für die Durchführung folgender Vorgänge sorgt: räumliche Definition zumindest des ersten Bildes (C1) des Eingriffsbereichs, in dem die zerstörende Behandlung durchgeführt werden soll, sowie die Darstellung einer Simulation der virtuellen Position zumindest eines der Emissionsköpfe (15) in diesem Eingriffsbereich auf dem zumindest einen ersten Bild (C1), das auf einem Monitor (101) angezeigt wird; wobei vorzugsweise das elektronische Programm (102) für die Darstellung auf dem mindestens einen Bild (C1, C2, C3) auf dem Monitor (101) mindestens einer virtuellen Bahn (K) vorsieht, die sich von der Halterungsposition des mindestens einen Emissionskopfes (15) an dem zweiten Halterungssystem (20) aus entwickelt, wobei die virtuelle Bahn (K) einen Weg für den Emissionskopf (15) in Richtung der virtuellen Position im Behandlungsbereich definiert.

8. Gerät (10) nach Anspruch 7, wobei das elektronische Programm (102) vorsieht, dass auf dem mindestens einen ersten Bild (C1), das auf einem Monitor (101) angezeigt wird, eine Simulation des Volumens (D) dargestellt wird, das von dem mindestens einen Emissionskopf (15) bestrahlt werden kann, der virtuell in dem Behandlungsbereich positioniert ist, und wobei die Größe des Volumens (D), das von dem mindestens einen, virtuell im Behandlungsbereich positionierten Emissionskopf (15) bestrahlt werden kann, eine Funktion eines oder mehrerer der folgenden Parameter ist: Leistung der emittierten Strahlung, Energiemenge oder Dosis der emittierten Strahlung, etwaige Bewegung des mindestens einen Emissionskopfes (15) während der Emissionsphase, die Länge dieser möglichen Bewegung, der Arrhenius-Schadenswert.

9. Gerät (10) nach den Ansprüchen 3 und 7, wobei die Simulationsvorgänge auch an dem zweiten Bild (C2) durchgeführt werden, das sich auf einen Scan der Sonde (12) in mindestens einer Ebene in Längsrichtung zur Hauptachse (Q) bezieht.

10. Gerät (10) nach einem oder mehreren der Ansprüche 7 bis 9, wobei das elektronische Programm (102) ein Modul (M3) zur destruktiven Behandlung des zu behandelnden Bereichs (30) umfasst, wobei das elektronische Programm (102) den Vorgang der Einstellung von Behandlungsparametern vorsieht, die den virtuellen Behandlungsparametern entsprechen, die im Gerät (10) während des Betriebs des Simulationsmoduls (M2) festgelegt sind, wobei diese Parameter eines oder mehrere der folgenden Elemente umfassen: die Anzahl der in der Emissionszone positionierten Emissionsköpfe (15), die Position des mindestens einen Emissionskopfes (15) in der Emissionszone, die Leistung der von dem mindestens einen Emissionskopf (15) emittierten Strahlung, die Energiemenge oder -dosis der von dem mindestens einen Emissionskopf (15) emittierten Strahlung, jede Bewegung des mindestens einen Emissionskopfes (15) während der Emissionsphase, die Länge der genannten möglichen Bewegung.

11. Gerät (10) nach einem oder mehreren der Ansprüche 7 bis 10, wobei das Simulationsmodul (M2) vorsieht
- das Berechnen in Echtzeit der Simulation auf der Grundlage eines oder mehrerer der folgenden Parameter: Anzahl der Ablationsköpfe (15), gegenseitige Positionierung der Ablationsköpfe (15), von jedem Ablationskopf (15) abgegebene Energiedosis, Leistung der Ablationsstrahlungsquelle (14), mögliches Vorhandensein von Rückzugsvorgängen mit Festlegung einer etwaigen Rückzugslänge/-distanz, oder
- eine Datenbank mit früheren Simulationen, die auf der Grundlage eines oder mehrerer der folgenden Parameter durchgeführt wurden: Anzahl der Ablationsköpfe (15), gegenseitige Positionierung der Ablationsköpfe (15), von jedem Ablationskopf (15) abgegebene Energiedosis, Leistung der Ablationsstrahlungsquelle (14), mögliches Vorhandensein von Rückzugsvorgängen mit Festlegung einer etwaigen Rückzugslänge/-distanz, sodass der Chirurg die Ergebnisse einer zuvor durchgeführten Simulation mit denselben oben angegebenen Betriebsparametern abrufen kann, wodurch Rechenzeit eingespart wird.

12. Verfahren zur Verarbeitung diagnostischer Bilder eines Körpervolumens, umfassend zumindest ein mittels einer Ultraschallsonde (12) aufgenommenes erstes Bild (C1), zur Simulation der Positionierung von Operationsnadeln (16) in einem zu behandelnden Bereich, wobei die Ultraschallsonde (12) eine Hauptachse (Q) zur Erfassung von Scans zumindest in einer zur Hauptachse (Q) quer (PT) und/oder längs verlaufenden Scanebene aufweist und wobei sich das erste Bild (C1) auf eine zur Achse (Q) der Sonde (12) quer verlaufende Ebene bezieht, wobei das Verfahren vorsieht
- das Erfassen der Drehpositionen (18A) der Sonde (12) um die Hauptachse (Q) in Echtzeit,
- das Anzeigen des mindestens einen ersten Querbildes (C1) auf einem Monitor (101), das sich auf einen Scan entlang einer der Hauptachse (Q) zugeordneten Scanebene bezieht,
- das Drehen des ersten Querbildes (C1) auf dem Monitor (101) in Übereinstimmung mit einer erfassten Drehbewegung der Sonde (12) um die Hauptachse (Q) derart, dass der Inhalt des ersten Bildes (C1) auf dem Monitor (101) fixiert bleibt.

13. Verfahren zur Verarbeitung diagnostischer Bilder nach dem vorstehenden Anspruch, das dazu ausgebildet ist, zumindest ein zweites Bild (C2) weiterzuverarbeiten, das sich auf einen Scan der Sonde (12) in mindestens einer zur Hauptachse (Q) längs verlaufenden Ebene bezieht, wobei sich das zweite Bild (C2) auf eine zur Achse (Q) der Sonde (12) längs verlaufende Ebene bezieht.

14. Verfahren zur Verarbeitung diagnostischer Bilder nach einem oder mehreren der vorstehenden Ansprüche, das vorsieht das Darstellen des Volumens (D), das von dem mindestens einen virtuellen Emissionskopf (15), der virtuell in dem Eingriffsbereich positioniert ist, bestrahlt werden kann.

15. Verfahren zur Verarbeitung diagnostischer Bilder nach einem oder mehreren der vorstehenden Ansprüche, wobei auf dem mindestens einen ersten Bild (C1) eine Umrissform einer den Interventionsbereich umgebenden Sicherheitszone (S1, S2, S3) sichtbar ist.

16. Verfahren zur Verarbeitung diagnostischer Bilder nach einem oder mehreren der vorstehenden Ansprüche, das vorsieht
- das Zuordnen einer virtuellen Positionierungsstruktur für mindestens einen virtuellen Strahlungsemissionskopf (15) zur Zerstörung von Tumorzellen zu dem mindestens einen ersten Bild (C1),
- das Anzeigen der virtuellen Positionierungsstruktur des virtuellen Emissionskopfes (15) auf dem mindestens einen ersten Bild (C1),
- das Sichtbarmachen einer Umrissform des Eingriffsbereichs (V1, V2, V3), in dem gewebezerstörende Strahlung emittiert werden soll, in dem mindestens einen ersten Bild (C1),
- das virtuelle Positionieren des virtuellen Strahlungskopfes (15) auf der virtuellen Positionierungsstruktur, um die zerstörende Strahlung virtuell in den Eingriffsbereich (V1, V2, V3) zu emittieren,
- das Überprüfen, ob das Volumen (D), das durch den mindestens einen virtuellen Strahlungskopf (15) bestrahlt werden kann, den gesamten Eingriffsbereich (V1, V2, V3) abdeckt.

## Revendications

1. Appareil médical (10) utilisant une approche ultrasonique pour des orifices naturels, comprenant :
• une sonde à ultrasons (12) apte à être introduite dans un orifice naturel d'un corps, ayant un axe principal (Q), configuré pour acquérir un volume spatial par acquisition de balayages le long d'au moins un plan de balayage associé audit axe principal (Q) ;
• un système de visionnage comprenant un moniteur (101) sur lequel au moins une première image (C1) relative à une image dans un plan perpendiculaire (PT) audit axe principal (Q) est visible ;
• un premier système de support (17) configuré pour supporter ladite sonde à ultrasons (12) dans la zone à examiner, lequel ledit système de support (17) étant apte à maintenir ladite sonde (12) en lui permettant deux mouvements, respectivement un premier mouvement axial dans lequel la sonde (12) est déplacée le long de son axe principal (Q) et un deuxième mouvement de rotation autour dudit axe principal (Q), la position de ladite sonde (12) dans ledit premier système de support (17) étant connue ;
• des indicateurs de position (18A, 18B) pour une position de rotation autour et une position le long dudit axe principal (Q) de ladite sonde (12) dans ledit premier système de support (17) ;
• au moins une aiguille de travail (16) apte à être introduite dans la partie du corps ;
• un deuxième système de support (20) configuré pour supporter ladite ou lesdites aiguille(s) de travail (16) et pour permettre son déplacement le long d'une trajectoire (K), vers la zone d'intervention dans laquelle elle(s) est(sont) apte(s) à œuvrer, ledit deuxième système de support (20) étant spatialement relié audit premier système de support (17) pour lequel la position de support de ladite aiguille de travail (16) est connue par rapport à ladite sonde à ultrasons (12) et par rapport au volume spatial examiné par ladite sonde à ultrasons (12) ;
• un dispositif électronique (100) pour commander l'appareil (10) ; **caractérisé en ce que** ledit dispositif électronique (100) comprend un module de correction de ladite ou desdites première(s) image(s) (C1) visible(s) sur le moniteur (101), apte à faire tourner ladite ou lesdites première(s) image(s) (C1) sur le moniteur (101) d'une manière correspondant au mouvement rotatoire de ladite sonde (12) autour dudit axe principal (Q), de sorte que le contenu de ladite ou lesdites première(s) image(s) relative(s) à la zone à traiter soit fixe sur le moniteur (101) durant la rotation de ladite sonde (12).

2. Appareil (10) selon la revendication 1, dans lequel ledit plan de balayage est :
• un plan perpendiculaire (PT) audit axe principal (Q) de la sonde (12), ladite ou lesdites première(s) image(s) (C1) relative à une image dans un plan perpendiculaire (PT) audit axe principal (Q) provenant d'au moins un balayage de ladite sonde (12) dans ledit plan (PT) perpendiculaire audit axe principal (Q), ou
• un plan longitudinal par rapport audit axe principal (Q), ladite ou lesdites première(s) image(s) (C1) relative(s) à une image dans un plan perpendiculaire (PT) audit axe principal (Q) étant reconstruite à partir d'une pluralité de balayages réalisés dans un plan longitudinal par rapport audit axe principal (Q).

3. Appareil (10) selon l'une ou plusieurs des revendications précédentes, dans lequel ledit moniteur (101) contient au moins une deuxième image enregistrée (C2) relative à un balayage de ladite sonde (12) dans au moins un plan longitudinal par rapport audit axe principal (Q).

4. Appareil (10) selon l'une ou plusieurs des revendications précédentes, dans lequel ladite sonde à ultrasons (12) comprend un corps oblong (12A) ayant une surface externe incurvée convexe s'étendant le long d'un développement longitudinal dudit corps (12A), ledit corps (12A) étant pourvu d'une pluralité de capteur ultrasoniques (12B) faisant face à la surface incurvée pour émettre et recevoir des ondes ultrasoniques ; de préférence ladite surface incurvée de ladite sonde à ultrasons (12) est sensiblement cylindrique et a un axe parallèle au développement longitudinal dudit corps oblong (12A) ; de préférence, les capteurs d'ultrasons (12B) de la sonde (12) sont agencés suivant au moins un rideau rectiligne (12B') parallèle à l'axe (Q) de la sonde (12), et au moins un rideau curviligne (12B") se situant sur une circonférence dans un plan perpendiculaire à l'axe (Q) de la sonde (12).

5. Appareil (10) selon l'une ou plusieurs des revendications précédentes, dans lequel ladite sonde à ultrasons (12) est une sonde à ultrasons endocavitaire (12), de préférence du type transrectal.

6. Appareil (10) selon l'une ou plusieurs des revendications précédentes, dans lequel ledit premier système de support (17) est apte à contraindre ladite sonde (12) pour permettre seulement deux mouvements : un premier mouvement axial dans lequel la sonde (12) est déplacée le long de son axe principal (Q) et un deuxième mouvement de rotation autour dudit axe principal (Q).

7. Appareil (10) selon l'une ou plusieurs des revendications précédentes, dans lequel ladite ou lesdites aiguille(s) de travail (16) est(sont) une(des) aguille(s) de guidage (16) mobile(s) le long d'une trajectoire (K) définie au moins en partie par ledit deuxième système de support (20) ; au moins une tête d'émission de rayonnement destructeur de tissu (15) étant de préférence agencée dans ladite ou lesdites aiguille(s) de guidage (16), ledit dispositif électronique (100) pour commander l'appareil (10) comprenant de préférence un programme électronique (102) ayant un module de simulation de destruction de tissu (M2) qui exécute des opérations de définition spatiale, sur ladite ou lesdites première(s) image(s) (C1) de la zone d'intervention où le traitement destructif doit être réalisé, l'affichage d'une simulation de la position virtuelle de ladite ou lesdites tête(s) d'émission (15) dans ladite zone d'intervention sur ladite ou lesdites première(s) image (s) (C1) représentée sur moniteur (101) ; de préférence ledit programme électronique (102) exécutant l'affichage sur ladite ou lesdites image(s) (C1, C2, C3) sur ledit moniteur (101), d'au moins une trajectoire virtuelle (K) se développant depuis la position de support pour ladite ou lesdites tête(s) d'émission (15) sur ledit deuxième système de support (20), ladite trajectoire virtuelle (K) définissant un chemin pour ladite tête d'émission (15) vers la position virtuelle dans la zone d'intervention.

8. Appareil (10) selon la revendication 7, dans lequel ledit programme électronique (102) exécute l'affichage, sur ladite ou lesdites première(s) image(s) (C1) représentée(s) sur un moniteur (101), d'une simulation du volume (D) qui peut être irradié par ladite ou lesdites tête(s) d'émission (15) positionnée(s) virtuellement dans ladite zone d'intervention, et dans lequel la taille du volume (D) qui peut être irradié par ladite ou lesdites tête(s) d'émission (15) positionné(s) virtuellement dans ladite zone d'intervention est fonction de l'un ou de plusieurs des paramètres suivants : la puissance du rayonnement émis, la quantité ou dose d'énergie du rayonnement émis, tout mouvement de ladite ou desdites tête(s) d'émission (15) durant la phase d'émission, la longueur dudit mouvement possible, la valeur du dommage d'Arrhenius.

9. Appareil (10) selon les revendications 3 et 7, dans lequel lesdites opérations de simulation sont également réalisées sur ladite deuxième image (C2) relative à un balayage de ladite sonde (12) dans au moins un plan longitudinal par rapport audit axe principal (Q).

10. Appareil (10) selon l'une ou plusieurs des revendications 7 à 9, dans lequel ledit programme électronique (102) comprend un module de traitement destructif (M3) de la zone à traiter, ledit programme électronique (102) exécutant l'opération de définition des paramètres de traitement correspondant à des paramètres de traitement virtuels définis dans l'appareil (10) durant l'opération dudit module de simulation (M2), lesdits paramètres comprenant l'un ou plusieurs des éléments suivants : le nombre de têtes d'émission (15) positionnées dans la zone d'émission, la position de la ou des tête(s) d'émission (15) dans la zone d'émission, la puissance du rayonnement émis par ladite ou lesdites tête(s) d'émission (15), la quantité ou la dose d'énergie du rayonnement émis par ladite ou lesdites tête(s) d'émission (15), tout mouvement de ladite ou desdites tête(s) d'émission (15) durant la phase d'émission, la longueur dudit mouvement possible.

11. Appareil (10) selon l'une ou plusieurs des revendications 7 à 10, dans lequel ledit module de simulation (M2) exécute :
• le calcul en temps réel de la simulation sur la base de l'un ou de plusieurs des paramètres suivants : le nombre de têtes d'ablation (15), le positionnement relatif des têtes d'ablation (15), la dose d'énergie appliquée par chaque tête d'ablation (15), la puissance de la source de rayonnement d'ablation (14), la présence possible de actions de repli, avec la définition de toute distance/longueur de repli, ou
• une base de données de simulations antérieurs réalisées dur la base de l'un ou de plusieurs des paramètre suivants : le nombre de têtes d'ablation (15), le positionnement relatif des têtes d'ablation (15), la dose d'énergie appliquée par chaque tête d'ablation (15), la puissance de la source de rayonnement d'ablation (14), la présence possible de actions de repli, avec la définition de toute longueur/distance de repli, de sorte que le chirurgien puisse recherche les résultats d'une simulation réalisée antérieurement avec les mêmes paramètres de fonctionnement que ceux indiqués ci-dessus, en vue d'économiser du temps de calcul.

12. Méthode de traitement d'images de diagnostic d'un volume corporel, incluant au moins une première image (C1) acquise au moyen d'une sonde à ultrasons (12), pour simuler le positionnement d'aiguilles de travail (16) dans une zone à traiter, dans lequel ladite sonde à ultrasons (12) comporte un axe principal (Q) pour l'acquisition de balayages au moins dans un plan de balayage perpendiculaire (PT) et/ou parallèle audit axe principal (Q), et dans laquelle ladite première image (C1) est relative à un plan perpendiculaire à l'axe (Q) de la sonde (12), ladite méthode exécutant :
- l'acquisition en temps réel des positions de rotation (18A) de ladite sonde (12) autour dudit axe principal (Q),
- l'affichage de ladite ou desdites première(s) image(s) transversale (C1) sur un moniteur (101) relative(s) à un balayage le long d'un plan de balayage associé avec ledit axe principal (Q),
- la rotation de ladite première image transversale (C1) sur le moniteur (101) d'une manière en concordance avec un mouvement de rotation acquis de ladite sonde (12) autour dudit axe principal (Q) de telle sorte que le contenu de ladite première image (C1) soit fixe sur le moniteur (101).

13. Méthode de traitement d'images de diagnostic selon la revendication précédente, apte en outre à traiter au moins une deuxième image (C2) relative à un balayage de ladite sonde (12) dans au moins un plan longitudinal par rapport audit axe principal (Q) et dans laquelle ladite deuxième image (C2) est relative à un plan longitudinal à l'axe (Q) de la sonde (12).

14. Méthode de traitement d'images de diagnostic selon l'une ou plusieurs des revendications précédentes, qui prévoit l'affichage du volume (D) qui peut être irradié par ladite ou lesdites tête(s) d'émission virtuelle (15) positionnée(s) virtuellement dans ladite zone d'intervention.

15. Méthode de traitement d'images de diagnostic selon l'une ou plusieurs des revendications précédentes, dans laquelle une forme de circonscription d'une zone de sureté (S1, S2, S3) entourant la zone d'intervention est visible sur ladite ou lesdites première(s) image(s) (C1).

16. Méthode de traitement d'images de diagnostic selon l'une ou plusieurs des revendications précédentes, qui exécute :
- l'association d'une structure de positionnement virtuel pour au moins une tête d'émission de radiation virtuelle (15) pour la destruction de cellules tumorales avec ladite ou lesdites première(s) image(s) (C1),
- l'affichage de ladite structure de positionnement virtuel de la tête d'émission de radiation virtuelle (15) sur ladite ou lesdites première(s) image(s) (C1),
- une forme de circonscription de la zone d'intervention (V1, V2, V3) où le rayonnement destructif de tissu doit être émis en étant visible sur ladite ou lesdites première(s) image(s) (C1),
- le positionnement virtuel de ladite tête d'émission de radiation virtuelle (15) sur ladite structure de positionnement virtuel de façon à émettre virtuellement le rayonnement destructif dans ladite zone d'intervention,
- la vérification que le volume (D) qui peut être irradié par ladite ou lesdites tête(s) d'émission de radiation virtuelle (15) couvre toute la zone d'intervention (V1, V2, V3).
